# EUROPÄISCHE PATENTSCHRIFT

(11) **EP 1 429 698 B1**
(45) Veröffentlichungstag und Bekanntmachung des Hinweises auf die Patenterteilung: **24.02.2010**
(21) Anmeldenummer: 02730161.3
(22) Anmeldetag: 16.04.2002
(51) Int. Cl.: A61F 13/00, B65H 3/08, B65H 39/10

(54) **GRUPPIERUNG VON FOLIEN- ODER BLATTARTIGEN MATERIALIEN**
GROUPING OF FILM-LIKE OR SHEET-LIKE MATERIALS
GROUPEMENT DE MATERIAUX SOUS FORME DE PELLICULES OU DE FEUILLES

(30) Priorität: 05.05.2001 DE 10121971
(43) Veröffentlichungstag der Anmeldung: 23.06.2004
(73) Patentinhaber: LTS LOHMANN Therapie-Systeme AG, 56626 Andernach (DE)
(72) Erfinder: THÖING, Heinrich, 53474 Bad Neuenahr-Ahrweiler (DE)
(74) Vertreter: Schmidt, Werner
(86) Internationale Anmeldenummer: PCT/EP2002/004179
(87) Internationale Veröffentlichungsnummer: WO 2002/089716

(56) Entgegenhaltungen:
- DE-A- 1 956 948
- DE-A- 4 400 713
- DE-C- 19 807 970
- GB-A- 843 631
- US-A- 2 298 363
- US-A- 5 941 681

## Beschreibung

Die Erfindung betrifft ein Verfahren zur Gruppierung einer Vielzahl von in einem Magazin gestapelten folien- oder blattartigen Materialien aus mindestens einer Materialschicht mit mindestens einem Sauger, der getaktet die folien- oder blattartigen Materialien ansaugt und aus dem Magazin entnimmt, wobei jeweils ein folien- oder blattartiges Material mit einem eine Anlagefläche bildenden Teil seiner Oberfläche an der Saugvorrichtung direkt anliegt.

Aus der DE 195 69 48 A1 ist ein Verfahren zur Trennung poröser Gegenstände wie Stoffmuster und zur Vereinzelung von Gegenständen mit starken Porositätsunterschieden bekannt, wobei jedes Teil einzeln mittels einer Saugvorrichtung von einem Stapel abgehoben wird.

Es sind Verfahren zur Vereinzelung von in einem Magazin gestapelten folien- oder blattartigen Materialien bekannt. Hierbei wird getaktet das einzelne folien- oder blattartige Material von einem Sauger aus dem Magazin entnommen und einzeln der Weiterverarbeitung zugeleitet.

Aus der US 2,298,363 A ist eine Verpackungseinheit mit einem einzelnen Pflaster bekannt. Es umfasst eine gefaltete Packstoffbahn und ein auf ein Trägermaterial aufgeklebtes Pflaster.

Sollen mehrere folien- oder blattartige Materialien gleichzeitig weiterverarbeitet werden, erfordert dies beispielsweise mehrere Magazine, in denen die verschiedenen folien- oder blattartige Materialien gelagert werden. Dies ist aufwendig und senkt die Geschwindigkeit der Weiterverarbeitung.

Der vorliegenden Erfindung liegt daher die Problemstellung zugrunde, ein Arbeitsverfahren zur Gruppierung von folien- oder blattartigen Materialien zu entwickeln, bei dem eine variable Anzahl von folien- oder blattartigen Materialien gruppiert werden kann, ohne die Geschwindigkeit der Weiterverarbeitung zu beeinträchtigen.

Diese Problemstellung wird mit den Merkmalen des Hauptanspruches gelöst. Dazu besteht ein Teil des Magazininhalts aus gasundurchlässigen folien- oder blattartigen Materialien, während ein anderer Teil folien- oder blattartige Materialien beinhaltet, die jeweils mit mindestens einer gasdurchlässigen Zone ausgestattet sind. Im Magazin folgt auf wenigstens ein gasdurchlässiges folien- oder blattartiges Material ein gasundurchlässiges folien- oder blattartiges Material. Außerdem liegt während des Ansaugens der folien- oder blattartigen Materialien durch den Sauger ein gasdurchlässiges folien- oder blattartiges Material mit seiner gasdurchlässigen Zone direkt am Sauger an und der Sauger zieht als entferntestes das nächste gestapelte gasundurchlässige folien- oder blattartige Material an.

Bei der Entnahme aus dem Magazin können mit dem Sauger mehrere folien- oder blattartiges Materialien zusammen entnommen werden und der Weiterverarbeitung zugeführt werden. Hierbei kann zum Beispiel ein gasdurchlässiges und ein gasundurchlässiges folien- oder blattartiges Material oder zum Beispiel auch zwei oder mehr gasdurchlässige und ein gasundurchlässige folien- oder blattartiges Material aus dem Magazin entnommen werden. Das gasundurchlässige folien- oder blattartige Material bildet hierbei immer das letzte bei einem Takt entnommene folien- oder blattartige Material. Das hier gasundurchlässige genannte folien- oder blattartige Material kann zumindest in einem gewissen Rahmen gasdurchlässig sein, wobei der dann in diesem Teil sich beim Ansaugen einstellende Gasstrom erheblich kleiner ist als jener Gasstrom, der sich beim Ansaugen durch das hier gasdurchlässig genannte folien- oder blattartige Material ergibt.

Die so entnommenen folien- oder blattartigen Materialien können gemeinsam der Weiterverarbeitung zugeführt werden. Die nachgeschalteten Arbeitsgänge müssen nicht auf das Zusammenstellen eines Loses warten. Somit wird die Geschwindigkeit der Weiterverarbeitung nicht durch die Anzahl der gemeinsam weiterverarbeiteten folien- oder blattartigen Materialien beeinträchtigt.

In einer nächsten Bearbeitungsstufe werden die folien- oder blattartigen Materialien dann beispielsweise einer Verpackungseinheit zugeführt. In diese Verpackungseinheit werden dann die bei einem Takt aus dem Magazin entnommenen zwei oder mehr folien- oder blattartigen Materialien verpackt. Das Material kann zum Beispiel einschichtig geformt sein oder aus mehreren, aufeinander aufgebrachten Schichten bestehen. Mindestens eine der in einer Verpackungseinheit verpackten folien- oder blattartigen Materialien enthält eine gasdurchlässige Zone. In mindestens einer anderen folien- oder blattartigem Material beträgt der Gasdurchlass maximal 50% des obengenannten Gasdurchlasses. Das letztgenannte folien- oder blattartige Material kann auch ohne Gasdurchlass gestaltet sein.

Weitere Einzelheiten der Erfindung ergeben sich aus den Unteransprüchen und der nachfolgenden Beschreibung eines schematisch dargestellten Ausführungsbeispiels.
- Figur 1:: Seitenansicht der Zuführung von folien- oder blattartigen Materialien aus einem Magazin an eine Verpackungsmaschine;
- Figur 2:: Detail des Entnahmebereichs des Magazins;
- Figur 3:: Seitenansicht der Zuführung der folien- oder blattartigen Materialien an ein Magazin über mehrere Arbeitsstationen;
- Figur 4:: Draufsicht der Figur 3.

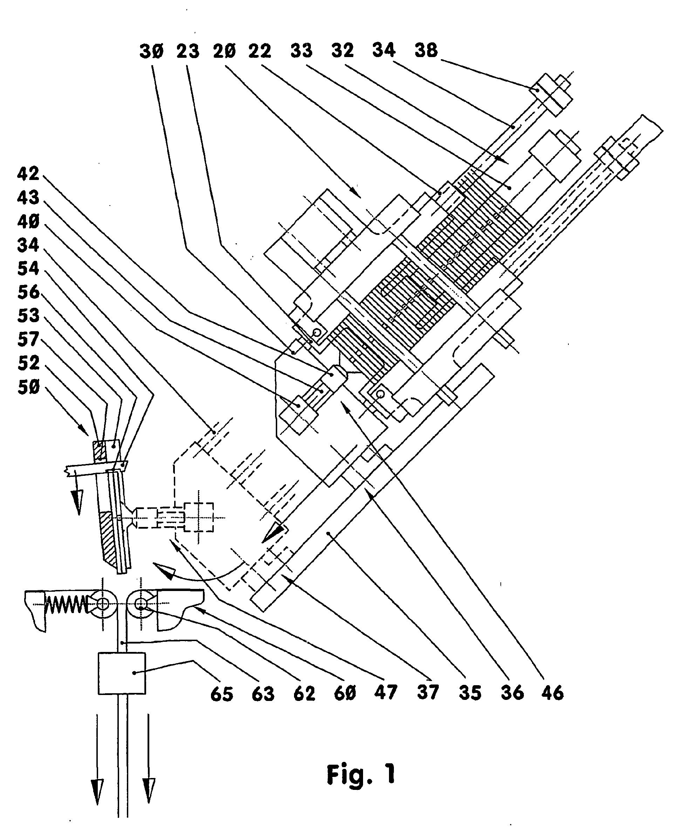

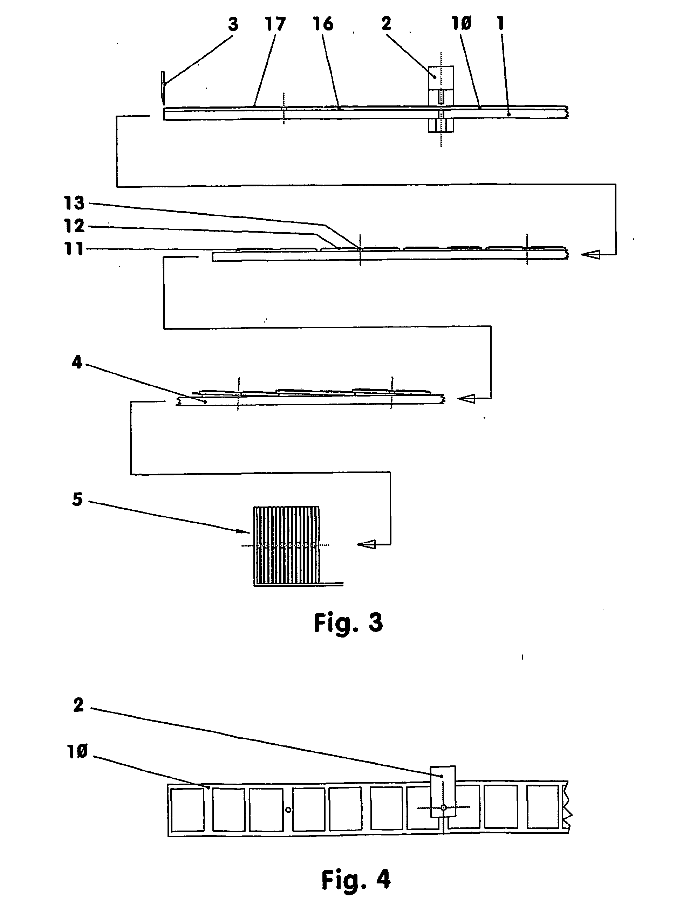

Die Figuren 3 und 4 zeigen das gruppenweise Vereinzeln, das Bearbeiten und die Zuführung von folien- oder blattartigen Materialien (11, 12) an ein Magazin (5, 20). Diese Materialien werden, da sie meist mehrschichtig sind, im Folgenden als Verbundmaterialfolien (11, 12) bezeichnet. Mit Hilfe einer Fördervorrichtung (1) wird eine Verbundmaterialfolienbahn (10) u.a. einer Lochstanze (2) und einer Schneidevorrichtung (3) zugeführt. An der Schneidvorrichtung (3) werden von der Verbundmaterialfolienbahn (10) einzelne, im allgemeinen gleichlange Verbundmaterialfolien (11, 12) abgeschnitten, die überlappend einem Schuppband (4) zugeführt werden. Die Verbundmaterialfolien (11, 12) werden nun gestapelt entweder über ein Vorratsmagazin (5) oder direkt dem in Figur 1 dargestellten Magazin (20) zugeführt.

Die Verbundmaterialfolienbahn (10), beispielsweise eine Pflasterbahn, umfasst z.B. eine Schutzfolie (16), beispielsweise einen Pflasterträger und auf diesen haftende einseitig beschichtete Wirkstoffträger (17), z.B. in Form von Pflastern. Die Pflasterbahn (10) hat hierbei beispielsweise die Breite eines Pflasters (17).

Die Pflaster (17) dienen beispielsweise als Therapiesysteme zur Übergabe von Wirkstoffen an die Haut eines Patienten. Sie sind z.B. rechteckig, elliptisch, rund, etc.. Auf ihrer Außenseite haben sie eine wirkstoffundurchlässige Trägermaterialschicht. Auf ihrer Beschichtungsseite ist z.B. mittig beispielsweise ein Vlies- oder Schaurnreservoir z.B. aus Baumwolle, Zellstoff, Polyethylen, Polyurethan etc. zur Wirkstoffaufnahme, z.B. von Lösungen, Emulsionen oder Suspensionen, angeordnet. An den äußeren Rändern der Pflaster (17) ist die Trägermaterialschicht zumindest bereichsweise auf der Beschichtungsseite mit einer Klebeschicht ausgestattet.

Die Pflaster (17) können beispielsweise auch Matrixsysteme auf Basis von Gemischen aus synthetischen Harzen, Elastomeren und Weichmachern oder natürlichen Proteinen, Poly- oder Disacchariden, synthetischen Wasserabsorbern, Feuchthaltemittel und ggf. natürlichen Klebstoffen sein.

In den verschiedenen Ausführungsformen der Pflaster (17) können ggf. auch Elemente zur Steuerung der Abgabe von Substanzen auf die Haut eines Patienten und zur Trennung einzelner Wirkstoffschichten eingelagert sein. Die Trägerfolie kann auch atmungsaktiv und dampfdurchlässig sein.

Der Pflasterträger (16) ist z.B. eine wirkstoffundurchlässige Schutzfolie. Er kann z.B. aus Papier, aus Polymerfolie, aluminiumbeschichteter Kunststofffolie, etc. bestehen. Der Pflasterträger (16) haftet ablösbar am Pflaster (17) und wird vor der Anwendung des Therapiesystems (17) von diesem entfernt. Er gibt dem Pflaster (17) vor der Anwendung Steifigkeit und verhindert den Austritt von Wirkstoffen aus dem Pflaster (17).

Zum Stanzen und z.B. gruppenweise Vereinzeln der Pflaster (17) wird die Pflasterbahn (10) von der Fördervorrichtung (1) so weit in Richtung der Schneidevorrichtung (3) transportiert, bis sie um einen voreingestellten Betrag, beispielsweise der Länge zweier Pflaster (17), über diese übersteht. Während des Abschneidens von Plasterstreifen (11, 12) von der Pflasterbahn (10) wird bei jedem zweiten Schnitt eine Lochstanze (2) betätigt, die z.B. einen Durchbruch (13) im Pflasterträger (16) zwischen zwei Pflastern (17) erzeugt. Nach jedem Schnitt wird die Pflasterbahn (10) weitergefördert und weitere Pflasterstreifen (11, 12) abgeschnitten.

Da der Durchbruch (13) nur während jedes zweiten Schnittes der Schneidevorrichtung (3) erzeugt wird, hat im Ausführungsbeispiel nur jeder zweite abgeschnittene Pflastersteifen (12) einen Durchbruch (13). Die Form des Durchbruchs (13) kann rund, eckig, elliptisch etc. sein. Er kann auch aus einer oder mehreren gasdurchlässigen Zonen (13) bestehen, die ebenfalls annähernd mittig und/oder in der Zone zwischen den Pflastern (17) auf dem Pflasterstreifen (12) angeordnet sind.

Durch ein entsprechendes Verhältnis zwischen Schnitten der Schneidevorrichtung (3) und Hüben der Lochstanze (2) kann die Anzahl der gelochten (12) zur Anzahl der ungelochten Pflasterstreifen (11) verändert werden. Werden beispielsweise während dreier aufeinanderfolgender Schnitte der Schneidevorrichtung (3) nur während der ersten beiden Hübe der Lochstanze (2) ausgeführt, folgen zwei gelochten Pflasterstreifen (12) ein ungelochter Pflasterstreifen (11).

Auf dem Schuppband (4) werden die einzelnen Pflasterstreifen (11, 12), die im Schnitt dargestellt sind, geordnet in der Reihenfolge ihres Abschneidens gestapelt, so dass die Pflasterstreifen (11, 12) aufeinanderliegen. So liegt hier auf jedem gelochten Pflasterstreifen (12) ein ungelochter Pflasterstreifen (11). Die gestapelten Pflasterstreifen (11, 12) können dann einem der Magazine (5, 20) zur Weiterverarbeitung zugeführt werden, vgl. Figur 1.

Die Figur 1 zeigt die Übergabe von Pflasterstreifen (11, 12) vom Magazin (20) an eine Verpackungsmaschine (60). An dem untersten der im Magazin (20) gestapelten Pflasterstreifen (11, 12) liegt ein auf einem Schlitten (30) schwenkbar gelagerter Flachsauger (42) an. Dieser entnimmt paketweise Pflasterträger (11, 12) aus dem Magazin (20).

Hiernach verfährt der Schlitten (30) den Flachsauger (42) mit den Pflasterstreifen (11, 12) an die Zuführung (50) einer Verpackungsmaschine (60). In letzterer werden die Pflasterstreifen (11, 12) verpackt.

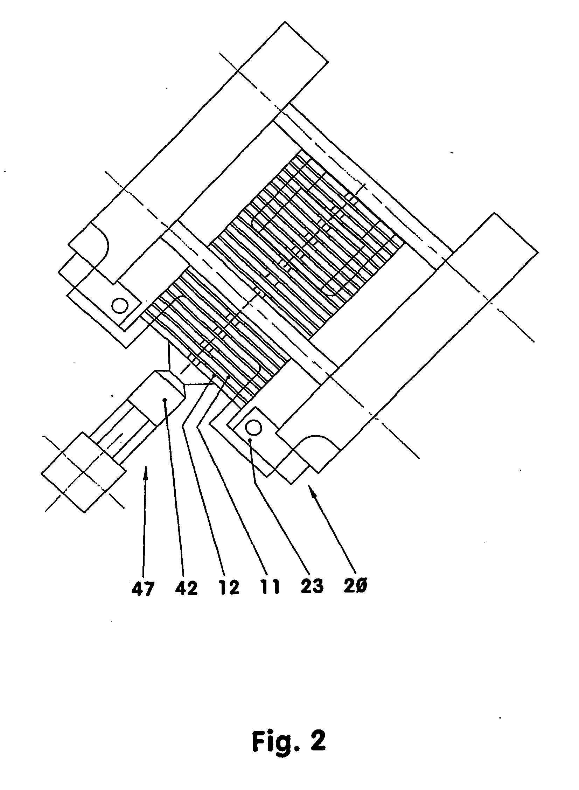

Das Magazin (20) ist beispielsweise unter einem Winkel von 45° zu Vertikalen angeordnet. Im Magazin (20), vgl. Figur 2, werden die Pflasterstreifen (11, 12) in Aufnahmeschienen (22) aufgenommen und geführt. Die Aufnahmeschienen (22) sind beispielsweise U-Profile, die an ihrem unteren Ende zumindest bereichsweise durch je ein Anlagestück (23) begrenzt sind.

Die zugeführten Pflasterstreifen (11, 12) liegen unter Einwirkung der Schwerkraft im Magazin (20). Der unterste Pflasterstreifen (12) liegt an den Anlagestücken (23) an. Die übrigen zugeführten Pflasterstreifen (11, 12) liegen dann auf diesem Pflasterstreifen (12) auf.

Der Schlitten (30) ist unterhalb des Magazins (20) in Normalrichtung zu den gestapelten Pflasterstreifen (11, 12) angeordnet. Er ist an zwei zueinander parallelen Führungsstangen (34) befestigt, die im Magazin (20) beispielsweise in Kugelbüchsen geführt sind. Unterhalb des Schlittens (30) ist eine Stützführung (35) angeordnet, an der das Magazin (20) angeordnet ist. Der Schlitten (30) wird durch eine pneumatische Zylinder-Kolben-Einheit (32) angetrieben, die beispielsweise mittig zwischen den Führungsstangen (34) angeordnet ist. Der Zylinder (33) der Zylinder-Kolben-Einheit (32) liegt auf der Höhe des Magazins (20). In einer oberen Endposition (36) des Schlittens (30) ist der Kolben der Zylinder-Kolben-Einheit (32) z.B. eingefahren, in einer unteren Endposition (37) ist der Kolben z.B. ausgefahren.

Der Schwenkkopf (40) ist beispielsweise ebenfalls pneumatisch betätigt. Er ist z.B. seitlich am Schlitten (30) gelagert. Seine Schwenkachse liegt quer zur Längsrichtung der Zylinder-Kolben-Einheit (32) und normal zur Darstellungsebene der Figur 1. Im Ausführungsbeispiel überstreicht der Schwenkkopf (40) einen Schwenkwinkel von etwa 225°.

Der Flachsauger (42) sitzt auf dem Schwenkkopf (40) und wird von diesem geschwenkt. In einer Entnahmeposition (46) steht die Mittelachse des Flachsaugers (42) in Stapelrichtung des Magazins (20) und normal zur Darstellungsebene der Figur 1. Ggf. ist der Saugdruck des Flachsaugers (42) variabel.

In der Übergabeposition (47) steht die Mittelachse des Flachsaugers (42) waagerecht etwa in Höhe der Mitte der Zuführung (50) an die Verpackungsmaschine (60).

Die Zuführung (50) hat eine Einschubplatte (52) mit seitlichen Führungsleisten (53), wobei die Einschubplatte (52) beispielsweise in einem Winkel von 5° zur Vertikalen angeordnet ist. Am oberen Ende der Zuführung (50) sind beispielsweise zwei in Langlöchern (57) der Einschubplatte (52) geführte Einschubstifte (54) angeordnet. In Figur 1 ist hiervon nur ein Einschubstift (54) dargestellt.

Der Flachsauger (42) liegt in der Entnahmeposition (46) mittig am untersten Pflasterstreifen (12) im Magazin (20) an. Durch Einschalten des Flachsauger (42) wird dieser Pflasterstreifen (12) durch den Flachsauger (42) angesaugt. Dieser Pflasterstreifen (12) hat in der Ansaugzone, in der der Pflasterstreifen (12) am Flachsauger (42) anliegt, einen Durchbruch (13), durch den sich das vom Flachsauger (42) erzeugte Vakuum bis zum nächsten Pflasterstreifen (11), der keinen Durchbruch (13) hat, ausdehnt. Hierbei ist der Saugstrom des Flachsaugers größer als die Summe aller Leckageströme, die ggf. zwischen den einzelnen beteiligten Pflasterstreifen (11, 12) entstehen. Der auf diesen gasundurchlässigen Pflasterstreifen (11) wirkende Luftdruck drückt alle zwischen dem Flachsauger (42) und diesem Pflasterstreifen (11) liegenden Pflasterstreifen (12) als Paket gegen den Flachsauger (42). Ggf. können auch die gasundurchlässigen Pflasterstreifen (11) im gewissen Rahmen gasdurchlässig gestaltet sein. Die Gasdurchlässigkeit dieser Pflasterstreifen (11) ist dann beispielsweise so gering, dass der beim Ansaugen des Flachsaugers (42) sich einstellende Gasstrom durch den gasundurchlässigen Pflasterstreifen (11) kleiner ist als der Gasstrom durch den gasdurchlässigen Pflasterstreifen (12).

Beim Wegfahren des Schlittens (30) in Richtung der Zuführung (50) bleibt der Unterdruck am Flachsauger (42) erhalten. Die Pflasterstreifen (11, 12) werden aus dem Magazin (20) herausgezogen. Die äußeren Enden der Pflasterstreifen (11, 12) rutschen hierbei von den Anlagestücken (23) herunter. Dabei wölben sich die Pflasterstreifen (11, 12) elastisch. Sobald die Pflasterstreifen (11, 12) aus dem Magazin (20) entnommen sind, geht die elastische Verformung wieder zurück.

Während der Schlitten (30) in seine untere Endposition (37) verfährt, schwenkt der Schwenkkopf (40) mit dem Flachsauger (42) und den an ihm anliegenden Pflasterstreifen (11, 12) im Uhrzeigersinn in Richtung der Übergabeposition (47). Ist die untere Endposition (37) des Schlittens (30) und die Übergabeposition (47) des Flachsaugers (42) erreicht, werden die Pflasterstreifen (11, 12) an die Zuführung (50) übergeben.

Hierbei werden die Pflasterstreifen (11, 12) an die Einschubplatte (52) der Zuführung (50) angelegt und an dieser entlang nach oben geschoben.

Liegen die Pflasterstreifen (11, 12) an der Einschubplatte (52) an, haften sie z. B. an der geneigten Einschubplatte (52). Gegen seitliches Verrutschen sind sie durch die Führungsleisten (53) gesichert.

Im einem nächsten Arbeitsschritt wird die Saugfunktion des Flachsaugers (42) abgeschaltet. Unmittelbar danach werden die Pflasterstreifen (11, 12) mit Hilfe der Einschubstifte (54) nach unten in Richtung der Verpackungsmaschine (60) verschoben.

Beispielsweise vor und/oder während des Abwärtsfahrens der Einschubstifte (54) schwenkt der Schwenkkopf (40) gegen den Uhrzeigersinn, bis der Flachsauger (42) wieder in Richtung des Magazins (20) zeigt. Nach Verfahren des Schlittens (30) in die obere Endposition (36) werden die nächsten Pflasterstreifen (11, 12) aus dem Magazin (20) entnommen. Sind nun beispielsweise die zwei folgenden Pflasterstreifen (12) mit einem Durchbruch (13) versehen, während der dritte Streifen (11) keinen Durchbruch (13) aufweist, wirkt die Sogwirkung durch die beiden gasdurchlässigen Pflasterstreifen (12) auf den gasundurchlässigen dritten Pflasterstreifen (11). Alle drei Streifen werden gemeinsam entnommen und über die Zuführung (50) der Verpackungsmaschine (60) zugeführt.

Die Verpackungsmaschine (60) umfasst zum Beispiel zwei Folienrollen, die im Verpackungsbereich als Packstoffbahnen (63) über je eine Leitrolle (62) geführt werden. Die beiden Leitrollen (62) begrenzen einen Spalt, der zwischen den Packstoffbahnen (63) enger ist als die Dicke eines üblichen Paketes aus Pflasterstreifen (11, 12). Eine der Leitrollen (62) ist normal zum Spalt beispielsweise federnd gelagert. Die beiden Packstoffbahnen (63) bilden je eine Außenseite der Verpackung und werden nach dem Einführen des Verpackungsgutes beispielsweise zu Siegelrand-, Peel- oder Schlauchbeuteln verschweißt. Ein Dickenmessgerät (65) prüft anschließend die Dicke des Verpackung.

An der Verpackungsmaschine (60) können die Pflasterstreifen (11, 12) aber beispielsweise auch in Tiefziehteile eingelegt werden.

Die zu verpackenden Pflasterstreifen (11, 12) werden von der Zuführung (50) aus von oben zwischen die Packstoffbahnen (63) im Bereich der Leitrollen (62) eingeführt. Hierbei werden die beiden Leitrollen (62) zumindest teilweise auseinandergedrückt. Nach dem Verschweißen z.B. der Vierrandsiegelbeutel wird am Dickenmessgerät (65) die Dicke der einzelnen Vierrandsiegelbeutels festgestellt. Die Dicke des Vierrandsiegelbeutels ist ein Maß für die Anzahl der im Beutel enthaltenen Pflasterstreifen (11, 12). Je nach Anzahl der Pflasterstreifen (11, 12) in einem Beutel kann dieser Beutel zur entsprechenden Weiterverarbeitungsstation oder zu einem Ausschussauswurf geleitet werden. Die Herstellung der gefüllten Vierrandsiegelbeutel kann so automatisiert mit wechselnder Anzahl der Pflasterstreifen (11, 12) pro Beutel erfolgen.

Ggf. können mit dem Verfahren auch einzelne Pflasterstreifen (11) verpackt werden. Dazu liegen im Magazin (20) viele gasundurchlässige Pflasterstreifen (11) übereinander. Am Dickenmessgerät (65) wird dann eine zu geringe Dicke des Vierrandsiegelbeutels als leerer Beutel interpretiert. Letzterer wird dann zum Ausschussauswurf geleitet.

### Bezugszeichenliste:

- 1: Fördervorrichtung
- 2: Lochstanze
- 3: Schneidevorrichtung
- 4: Schuppband
- 5: Vorratsmagazin

- 10: Verbundmaterialfölienbahn, Pflasterbahn
- 11: folien- oder blattartige Materialien, gasundurchlässig; Verbundmaterialfolien, gasundurchlässig; Pflasterstreifen, ungelocht
- 12: folien- oder blattartige Materialien, gasdurchlässig; Verbundmaterialfolien, gasdurchlässig; Pflasterstreifen, gelocht
- 13: Durchbruch in (12), gasdurchlässige Zonen in (12)

- 16: Schutzfolie, Pflasterträger
- 17: Wirkstoffträger, Pflaster, Therapiesystem

- 20: Magazin

- 22: Aufnahmeschienen
- 23: Anlagestücke

- 30: Schlitten

- 32: Zylinder-Kolben-Einheit
- 33: Zylinder
- 34: Führungsstangen
- 35: Stützführung
- 36: obere Endposition
- 37: untere Endposition
- 38: Anschläge

- 40: Schwenkkopf

- 42: Sauger, Flachsauger
- 43: Mittelachse

- 46: Entnahmeposition
- 47: Übergabeposition

- 50: Zuführung

- 52: Einschubplatte
- 53: Führungsleisten
- 54: Einschubstifte

- 56: Aussparung
- 57: Langlöcher

- 60: Verpackungsmaschine

- 62: Leitrollen
- 63: Packstoffbahnen

- 65: Dickenmessgerät

## Patentansprüche

1. Verfahren zur Gruppierung einer Vielzahl von in einem Magazin gestapelten folien- oder blattartigen Materialien aus mindestens einer Materialschicht mit mindestens einem Sauger, der getaktet die folien- oder blattartigen Materialien ansaugt und aus dem Magazin entnimmt, wobei jeweils ein folien- oder blattartiges Material mit einem eine Anlagefläche bildenden Teil seiner Oberfläche an der Saugvorrichtung direkt anliegt, **dadurch gekennzeichnet,**
- **dass** ein Teil des Magazininhalts aus gasundurchlässigen folien- oder blattartigen Materialien (11) besteht, während ein anderer Teil folien- oder blattartige Materialien (12) beinhaltet, die jeweils mit mindestens einer gasdurchlässigen Zone (13) ausgestattet sind,
- **dass** im Magazin (20) auf wenigstens ein gasdurchlässiges folien- oder blattartiges Material (12) einem gasundurchlässiges folien- oder blattartiges Material (11) folgt, und
- **dass** während des Ansaugens der folien- oder blattartigen Materialien (11, 12) durch den Sauger (42) ein gasdurchlässiges folien- oder blattartiges Material (12) mit seiner gasdurchlässigen Zone (13) direkt am Sauger (42) anliegt und der Sauger (42) als entferntestes das nächste gestapelte gasundurchlässige folien- oder blattartige Material (11) anzieht.

2. Verfahren nach Anspruch 1, **dadurch gekennzeichnet, dass** eine gasdurchlässige Zone (13) ein Durchbruch in Form eines einzelnen, größeren Loches ist oder eine Summe mehrerer, kleinerer Löcher darstellt, wobei die Querschnittsfläche des einzelnen, größeren Loches zumindest annähernd gleich der Querschnittsfläche der Summe aller kleineren Löcher ist.

3. Verfahren nach Anspruch 2, **dadurch gekennzeichnet, dass** der Durchbruch (13) in der Nähe der Mitte des folien- oder blattartigen Materials (12) angeordnet list.

4. Verfahren nach Anspruch 1, **dadurch gekennzeichnet, dass** die folien- oder blattartigen Materialien (11, 12) Pflasterstreifen sind.

5. Verfahren nach Anspruch 4, **dadurch gekennzeichnet, dass** die Pflasterstreifen (11, 12) mehrere Pflaster (17) umfassen.

6. Verfahren nach Anspruch 1, **dadurch gekennzeichnet, dass** der Sauger (42) ein Flachsauger (42) ist.

7. Verfahren nach Anspruch 1, **dadurch gekennzeichnet, dass** der Druck des Saugers (42) einstellbar ist.

8. Verfahren nach Anspruch 2, **dadurch gekennzeichnet, dass** dem Magazin (20) eine Vorrichtung zur Erzeugung der Durchbrüche (13) vorgeschaltet ist.

9. Verfahren nach Anspruch 1, **dadurch gekennzeichnet, dass** dem Magazin (20) eine Vorrichtung zur Verpackung der entnommenen folien- oder blattartigen Materialien (11, 12) nachgeschaltet ist.

10. Verpackungseinheit mit zwei Packstoffbahnen (63) und mit mindestens zwei zwischen diesen angeordneten folien- oder blattartige Materialien (11, 12), wobei die folien- oder blattartige Materialien (11, 12) jeweils aus mindestens einer Materialschicht bestehen, **dadurch gekennzeichnet, dass** mindestens eines der folien- oder blattartige Materialien (12) eine gasdurchlässige Zone (13) enthält, während ein anderes folien- oder blattartiges Material (11) entweder keine gasdurchlässige Zone (13) hat oder mit einer gasdurchlässigen Zone ausgestattet ist, deren Gasdurchlass um mindestens 50% kleiner ist als der Gasdurchlass des erstgenannten Materials (12).

11. Verpackungseinheit nach Anspruch 10, **dadurch gekennzeichnet**, das die Verpackung dieser Einheit ein Vierrandsiegelbeutel ist.

## Claims

1. Method for grouping a multiplicity of film-like or sheet-like materials which are stacked in a magazine and which consist of at least one material layer, using at least one suction apparatus which suctions the film-like or sheet-like materials in a clocked manner and removes them from the magazine, one film-like or sheet-like material in each case resting directly with a portion of its surface, forming a contact surface, against the suction device, **characterized in that**
- one portion of the magazine content consists of gas-impermeable film-like or sheet-like materials (11), while another portion contains film-like or sheet-like materials (12) each provided with at least one gas-permeable zone (13),
- in the magazine (20), at least one gas-permeable film-like or sheet-like material (12) is followed by a gas-impermeable film-like or sheet-like material (11), and
- during the suctioning of the film-like or sheet-like materials (11, 12) by the suction apparatus (42), one gas-permeable film-like or sheet-like material (12) lies with its gas-permeable zone (13) directly on the suction apparatus (42), and the suction apparatus (42) takes up the next farthest away stacked gas-impermeable film-like or sheet-like material (11).

2. Method according to Claim 1, **characterized in that** a gas-permeable zone (13) is an interruption in the form of a large individual hole or represents a sum of several smaller holes, the cross-sectional area of the large individual hole being at least approximately equal to the cross-sectional area of the sum of all the smaller holes.

3. Method according to Claim 2, **characterized in that** the interruption (13) is arranged near the center of the film-like or sheet-like material (12).

4. Method according to Claim 1, **characterized in that** the film-like or sheet-like materials (11, 12) are plaster strips.

5. Method according to Claim 4, **characterized in that** the plaster strips (11, 12) comprise several plasters (17).

6. Method according to Claim 1, **characterized in that** the suction apparatus (42) is a flat suction apparatus (42).

7. Method according to Claim 1, **characterized in that** the pressure of the suction apparatus (42) is adjustable.

8. Method according to Claim 2, **characterized in that** a device for producing the interruptions (13) is placed upstream of the magazine (20).

9. Method according to Claim 1, **characterized in that** a device for packaging the removed film-like or sheet-like materials (11, 12) is placed downstream of the magazine (20).

10. Packaging unit having two webs of packaging material (63) and having at least two film-like or sheet-like materials (11, 12) arranged between them, the film-like or sheet-like materials (11, 12) each consisting of at least one material layer, **characterized in that** at least one of the film-like or sheet-like materials (12) contains a gas-permeable zone (13), while another film-like or sheet-like material (11) either has no gas-permeable zone (13) or is provided with a gas-permeable zone whose gas permeability is at least 50% less than the gas permeability of the first-mentioned material (12).

11. Packaging unit according to claim 10, **characterized in that** the package from this unit is a rectangular sealed bag.

## Revendications

1. Procédé pour le groupement d'une pluralité de matériaux sous forme de pellicules ou de feuilles empilés dans un magasin, constitués d'au moins une couche de matériau, comprenant au moins un dispositif d'aspiration qui aspire de manière cadencée les matériaux sous forme de pellicules ou de feuilles et qui les prélève du magasin, un matériau sous forme de pellicule ou de feuille s'appliquant à chaque fois directement contre le dispositif d'aspiration avec une partie de sa surface formant une surface d'appui,
**caractérisé en ce que**
- une partie du contenu du magasin se compose de matériaux sous forme de pellicules ou de feuilles (11) imperméables aux gaz, tandis qu'une autre partie contient des matériaux sous forme de pellicules ou feuilles (12) qui sont pourvus à chaque fois d'au moins une zone perméable aux gaz (13),
- dans le magasin (20), au moins un matériau sous forme de pellicule ou de feuille (12) perméable aux gaz est suivi d'un matériau sous forme de pellicule ou de feuille (11) imperméable aux gaz, et
- pendant l'aspiration des matériaux sous forme de pellicules ou de feuilles (11, 12) par le dispositif d'aspiration (42), un matériau sous forme de pellicule ou de feuille (12) perméable aux gaz s'applique avec sa zone perméable aux gaz (13) directement contre le dispositif d'aspiration (42) et le dispositif d'aspiration (42) attire le matériau sous forme de pellicule ou de feuille (11) imperméable aux gaz empilé à la suite, en tant que matériau le plus éloigné.

2. Procédé selon la revendication 1, **caractérisé en ce qu'**une zone perméable aux gaz (13) est un orifice en forme de trou unique relativement grand, ou est constituée d'une somme de plusieurs trous plus petits, la surface en section transversale du trou unique plus grand étant au moins approximativement égale à la surface en section transversale de la somme de tous les trous plus petits.

3. Procédé selon la revendication 2, **caractérisé en ce que** l'orifice (13) est disposé à proximité du centre du matériau sous forme de pellicule ou de feuille (12).

4. Procédé selon la revendication 1, **caractérisé en ce que** les matériaux sous forme de pellicules ou de feuilles (11, 12) sont des bandes de pansement.

5. Procédé selon la revendication 4, **caractérisé en ce que** les bandes de pansement (11 ,12) comprennent plusieurs pansements (17).

6. Procédé selon la revendication 1, **caractérisé en ce que** le dispositif d'aspiration (42) est un dispositif d'aspiration à plat (42).

7. Procédé selon la revendication 1, **caractérisé en ce que** la pression du dispositif d'aspiration (42) peut être ajustée.

8. Procédé selon la revendication 2, **caractérisé en ce que** l'on monte en amont du magasin (20) un dispositif pour produire les orifices (13).

9. Procédé selon la revendication 1, **caractérisé en ce que** l'on monte en aval du magasin (20) un dispositif pour l'emballage des matériaux sous forme de pellicules ou de feuilles (11, 12) prélevés.

10. Unité d'emballage comprenant deux bandes de matériau d'emballage (63) et au moins deux matériaux sous forme de pellicules ou de feuilles (11, 12) disposés entre celles-ci, les matériaux sous forme de pellicules ou de feuilles (11, 12) se composant à chaque fois d'au moins une couche de matériau, **caractérisée en ce qu'**au moins l'un des matériaux sous forme de pellicules ou de feuilles (12) contient une zone perméable aux gaz (13), tandis qu'un autre matériau sous forme de pellicule ou de feuille (11) n'a aucune zone perméable aux gaz (13) ou bien est pourvu d'une zone perméable aux gaz dont la perméabilité aux gaz est au moins inférieure de 50% à la perméabilité aux gaz du premier matériau (12).

11. Unité d'emballage selon la revendication 10, **caractérisée en ce que** l'emballage de cette unité est un sac scellable à quatre bords.
